# EUROPEAN PATENT SPECIFICATION

(11) **EP 3 326 696 B1**
(45) Date of publication and mention of the grant of the patent: **10.04.2019**
(21) Application number: 17204115.4
(22) Date of filing: 28.11.2017
(51) Int. Cl.: A61Q 19/10, A45D 40/24, A61K 8/02, C11D 17/04

(54) **CLICK TOGETHER" SOLID CLEANSING COMPOSITION**
ZUSAMMENKLICKBARE, FESTE REINIGUNGSZUSAMMENSETZUNG
COMPOSITION NETTOYANTE SOLIDE «À CLIQUER ENSEMBLE »

(30) Priority: 29.11.2016 US 201615363722
(43) Date of publication of application: 30.05.2018
(73) Proprietor: Henkel IP & Holding GmbH, 40589 Düsseldorf (DE)
(72) Inventor: KHAMIS, Dan, AZ 85022, Phoenix, (US); CONWAY, Mary J, AZ 85022, Phoenix, (US); SALAS, Juan, Arizona 85213, Mesa, (US)
(74) Representative: Henkel IP Department

(56) References cited:
- FR-A1- 2 364 268
- US-A- 4 177 965
- US-A1- 2006 011 569
- US-A1- 2015 057 215

## Description

The present invention generally relates to solid cleansing compositions, and more particularly relates to an apparatus and method for combining solid cleansing compositions according to a user's preferences.

Humans have been making soap since at least the times of ancient Babylon. In the almost five millennia since humans started making soap, the soap has taken a variety of forms and been used for a variety of purposes, most of which have been cleansing compositions.

Soap bars are cleansing compositions that are in solid form and as a consequence, do not require containers, cannot be spilled, and are used incrementally. During use, an outer layer of a soap bar is removed while leaving the layers underneath unaffected.

Solid cleansing compositions, such as soap bars, are provided with a variety of perfumes, moisturizing agents, skin conditioners, exfoliants, plant matter, and a number of other additives. These additives cater the solid cleansing composition to the moisture of a user's skin, olfactory preferences, the oil production rate of a user's skin, and other such user-specific properties.

US 2015/0057215 A1 discloses a soap kit comprising a plurality of soap pieces for assembly, each one of said soap pieces being configures to engage with a geometrically conforming portion of at least another complementary one of said soap pieces, such that said soap pieces can be collectively arranged to form an integrated soap unit. The soap pieces can have different fragrances or different hygienic aim (shampoo, hair conditioner, skin moisturiser) or different colours and the consumer can the assemble soap units depending on the desired characteristics.

Accordingly, it is desirable to provide a solid cleansing composition that may be used to cleanse a skin surface. In addition, it is desirable to provide a solid cleansing composition that conforms to a user's preferences, by allowing a user to select specific attributes of a solid cleansing composition to incorporate into a composite solid cleansing composition.

An apparatus is provided for a user-customizable solid cleansing composition. The apparatus comprises at least two solid cleansing compositions, each of which includes at least one surfactant and at least one attachment module, wherein the attachment modules fit together so as to assemble the at least two solid cleansing compositions to a composite composition. At least one of the attachment modules of the composite solid cleansing composition is the same as a conjugate attachment module. Alternatively one of the attachment modules of the composite solid cleansing composition is different from a conjugate attachment module. Further an attachment module of the composite solid cleansing composition is alternatively detachable or not detachable from a conjugate attachment module.

A method is provided for assembling a composite solid cleansing composition. The method comprises providing at least two solid cleansing compositions, each of which comprises an attachment module, and attaching the at least two solid cleansing compositions through their attachment modules.

A method is providing for making a solid cleansing composition to become part of a composite solid cleansing composition. The method comprises providing an attachment module that includes a first part that includes a contact point to another attachment module, and a second part that anchors the attachment module in the solid cleansing composition, and forming a solid cleansing composition around the second part of the attachment module.

The present invention will hereinafter be described in conjunction with the following drawing figures, wherein like numerals denote like elements, and
FIG. 1 is a diagram of a composite solid cleansing composition comprising two component solid cleansing compositions, according to an example of the principles described herein;
FIG. 2 is a diagram of a composite solid cleansing composition comprising three component solid cleansing compositions, according to an example of the principles described herein;
FIG. 3A-3C are diagrams of two conjugate attachment modules, in the pre-attachment (FIGS. 3A and 3B) and post-attachment (FIG. 3C) states, according to an example of the principles described herein;
FIG. 4A-4C are diagrams of two conjugate attachment modules, in the pre-attachment (FIGS. 4A and 4B) and post-attachment (FIG. 4C) states, according to an example of the principles described herein;
FIG. 5A-5C are diagrams of two conjugate attachment modules, in the pre-attachment (FIGS. 5A and 5B) and post-attachment (FIG. 5C) states, according to an example of the principles described herein;
FIG. 6A-6C are diagrams of two conjugate attachment modules, in the pre-attachment (FIGS. 6A and 6B) and post-attachment (FIG. 6C) states, according to an example of the principles described herein;
FIG. 7 is a diagram of a part of an attachment module that anchors the attachment module into a solid cleansing composition, according to an example of the principles described herein;

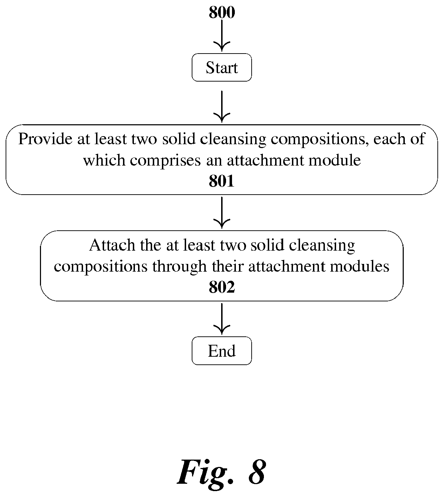

FIG. 8 is a flowchart of a method for making a composite solid cleansing composition, according to an example of the principles described herein; and

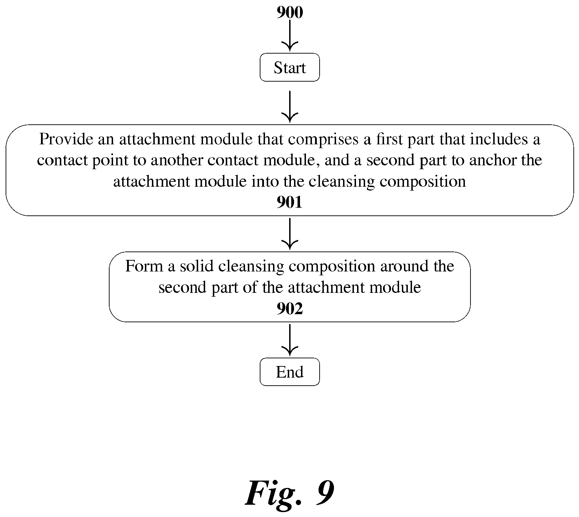

FIG. 9 is a flowchart of a method for making a solid cleansing composition to become part of a composite solid cleansing composition, according to an example of the principles described herein.

The following detailed description of the invention is merely exemplary in nature and is not intended to limit the invention or the application and uses of the invention. Furthermore, there is no intention to be bound by any theory presented in the preceding background of the invention or the following detailed description of the invention.

As used herein, the term "about" refers to amounts, concentrations or values that are within 10 percent of the expressed amount, concentration or value.

As mentioned above, solid cleansing compositions are used by individuals to cleanse their skin. Individuals may have distinct preferences as to what elements of a solid cleansing composition they would like included in a solid cleansing composition that they use to cleanse their skin. Existing solid cleansing compositions may be configured to be used for a single purpose, and thus may not allow a user to customize the solid cleansing composition to their own preferences regarding such aspects as surfactant composition, moisturizing ingredients, perfume, exfoliants, acne treatment agents, and the like.

Accordingly, the present specification is directed to a way to allow a user to customize a solid cleansing composition to conform with their preferences. The present specification provides for a solid cleansing composition that may be combined with at least one other solid cleansing composition to produce a composite solid cleansing composition. The composite solid cleansing composition may provide each of the properties and benefits of the component solid cleansing compositions.

According to one example of the present specification, a user may assemble a solid cleansing composition that includes at least one moisturizing ingredient and at least one acne treatment agent. In another example of the present specification, a user may assemble a solid cleansing composition that includes two distinct perfumes. In a further example of the present specification, a user may assemble a solid cleansing composition that includes an amount of a moisturizing ingredient that is between the amount provided in either of the component solid cleansing compositions. In a still further example of the present specification, a user may assemble a solid cleansing composition that contains an acne treatment agent, an exfoliant, and an antibacterial agent. In principle, any combination is possible, and may be achieved according to a user's preferences.

The present specification also includes a method of making a composite solid cleansing composition. The method includes providing at least two solid cleansing compositions, each of which includes an attachment module. The method further includes attaching the at least two solid cleansing compositions through their attachment modules.

The present specification also includes a method of making a solid cleansing composition to become part of a composite cleansing composition that includes providing an attachment module that includes a first part and a second part. The first part includes a contact point to contact another attachment module; the second part includes an anchor that anchors the attachment module to the solid cleansing composition. The method also includes forming a solid cleansing composition around the second part of the attachment module.

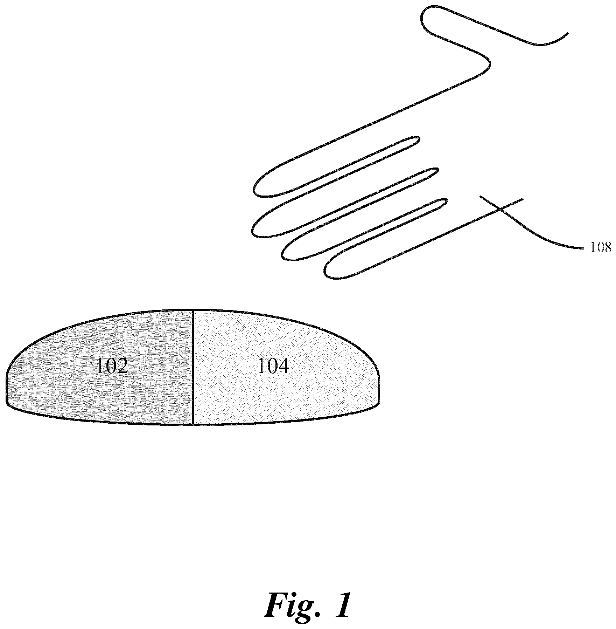

Turning now to the figures, Fig. 1 shows a composite solid cleansing composition according to an example of the present specification. The composite solid cleansing composition shown in Fig. 1 includes two solid cleansing compositions. The first solid cleansing composition (102) may have a different composition from the second solid cleansing composition (104). Fig. 1 shows a tight seam where the first solid cleansing composition (102) fits together with the second solid cleansing composition (104). When a user applies the composite solid cleansing composition shown in Fig. 1 to the user's hands (108), the outer layer of the composite solid cleansing composition is applied to the user's hands (108), which includes both the first solid cleansing composition (102) and the second solid cleansing composition (104).

In Fig. 1, the first solid cleansing composition (102) fits together with the second solid cleansing composition (104) in a manner that encloses the attachment modules that mediate the attachment between the first solid cleansing composition (102) and the second solid cleansing composition (104). As a result, in Fig. 1, the attachment module is contained within the composite solid cleansing composition, and is not on the outer surface of the composite solid cleansing composition. Enclosing the attachment module within the composite solid cleansing composition may ensure that the entire outer surface is the composite solid cleansing composition, to provide a user with a conventional feel when using the solid cleansing composition. It is also possible according to the present specification that the attachment module is at least partially located on an outer surface of the cleansing composition. Locating the attachment module adjacent to an outer surface of the composite solid cleansing composition may provide a surface on which to rest the composite solid cleansing composition, whereby preventing the composite solid cleansing composition from becoming slimy or developing a film from extended contact with water.

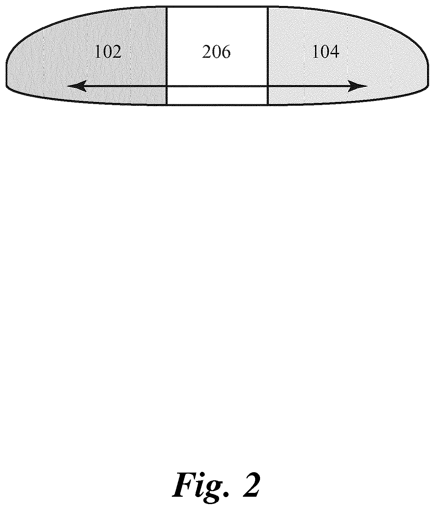

Fig. 2 depicts a three-part composite solid cleansing composition, according to an example of the present specification. A central solid cleansing composition (206) is fit together with both a first solid cleansing composition (102) and a second solid cleansing composition (104). In the example shown in Fig. 2, only a single central solid cleansing composition (206) is used; however, it is also possible to use more than one central solid cleansing composition (206), which may be either the same or different from one another. Fig. 2 also shows the attachment axis, which is provided as a line running from the first solid cleansing composition (102) to the second solid cleansing composition (104). In Fig. 2, the boundaries between each solid cleansing composition (102, 206, and 104) are orthogonal to the attachment axis; however, the boundaries between each solid cleansing composition may also be oblique to the attachment axis. The attachment axis may be defined individually for each interface between solid cleansing compositions according to the present specification, and if there is more than one attachment axis, the attachment axes may be parallel, or may be not parallel. For example, a number of solid cleansing compositions may be provided so that the composite solid cleansing composition is in the shape of a disc, torus, or any other suitable shape.

In Figs. 1 and 2, the first solid cleansing composition (102) and the second solid cleansing composition (104) are distinct from one another, and also distinct from a central solid cleansing composition (206). It is also possible that the composite solid cleansing composition may include the same composition for two or more of the component solid cleansing compositions. For example, a first solid cleansing composition (102) and a second solid cleansing composition (104) may be the same, and may optionally include a central solid cleansing composition (206) which is distinct. In another example, a first solid cleansing composition (102) is the same as the central solid cleansing composition (206), and distinct from a second solid cleansing composition (104). Such an arrangement may be provided in order to modulate the ratios of the components in each cleansing composition.

Each solid cleansing composition (102/104/206) may have the same or different base composition, and may include the same or different perfumes, adjuvants and/or benefit agents. The components of the solid cleansing compositions are discussed further below.

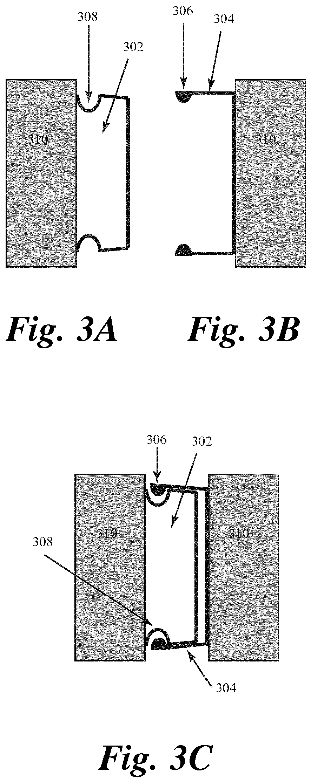

Figs. 3-6 depict examples of four attachment module types, emphasizing a first part of each attachment module that includes a contact point to interact with another attachment module. For an attachment module, the attachment module with which it fits together is referred to herein as its conjugate attachment module. An attachment module and its conjugate attachment module together may be referred to as a conjugate pair of attachment modules. Each of Fig. 3-6 shows the pre-attachment state of a conjugate pair of attachment modules (A and B) and the post-attachment state (C), according to examples of the present specification. Each attachment module shown in Figs. 3-6 includes a first part that provides the contact point to a conjugate attachment module; each attachment module also includes a second part (310) that anchors the attachment module in a solid cleansing composition.

Figs. 3A and 3B depict a conjugate pair of attachment modules that attach to one another using an outer clamp type attachment mechanism. As the two attachment modules shown in Figs. 3A and 3B are pushed toward one another, the guiding element (304) of the attachment module shown in Fig. 3B flexes outward around the structural element (302) of the attachment module shown in Fig. 3A. In Fig. 3A, the structural element (302) is equipped with a locking element (308), which in Fig. 3A takes the form of a divot. The guiding element (304) of the attachment module shown in Fig. 3B is equipped with a locking element (306), which in Fig. 3B takes the form of a protrusion on the guiding element (304).

Fig. 3C depicts the outer clamp type attachment mechanism of Figs. 3A and 3B in the post-attachment state. Fig. 3C shows that in the post-attachment state, the protrusion-type locking element (306) sits in the divot-type locking element (308). In Fig. 3C, the guiding element (304) wraps around the structural element (302).

Analogous to the diagrams shown in Fig. 3, an inner clamp type mechanism may also be used, in which the guiding elements (304) flex inward, toward one another.

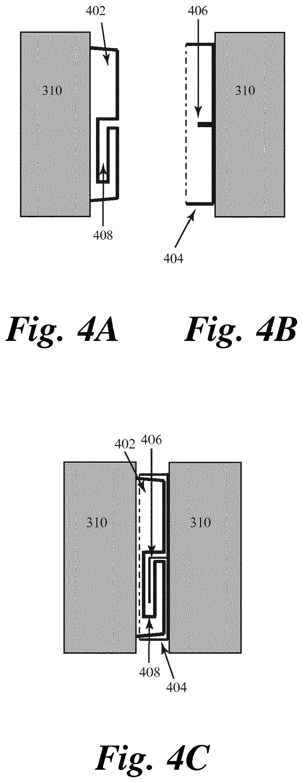

Figs. 4A and 4B depict a conjugate pair of attachment modules using a rotating-tabs type attachment mechanism. The attachment module shown in Fig. 4A includes an indentation type locking element (408) that is set in the structural element (402) of the attachment module. The attachment module shown in Fig. 4B includes a guiding element (404). The guiding element (404) may be circular or otherwise configured to allow the attachment module shown in Fig. 4B to rotate along an axis that is horizontal and in the plane of the page, e.g., running from Fig. 4A to Fig. 4B. The parts of the guiding element (404) not in the plane of the cross-section of Fig. 4B are shown by the dashed line. Fig. 4B also features an L-shaped locking element (406). In Fig. 4B, the L-shaped locking element (406) is directed out of the page. The attachment modules of Fig. 4A and 4B are attached by moving the modules toward one another until the L-shaped locking element (406) slides into an indentation (408) on the conjugate attachment module. The conjugate attachment modules are then twisted relative to one another to firmly hold the attachment modules in their post-attachment states.

Fig. 4C depicts the rotating-tabs type attachment mechanism of Figs. 4A and 4B in the post-attachment state. Fig. 4C shows that in the post-attachment state, the attachment module of Fig. 4B has been rotated, relative to the position of the attachment module of Fig. 4A. In Fig. 4C, the L-shaped locking element (406) extends into the indentation type locking element (408), to firmly hold the two attachment modules in the post-attachment state.

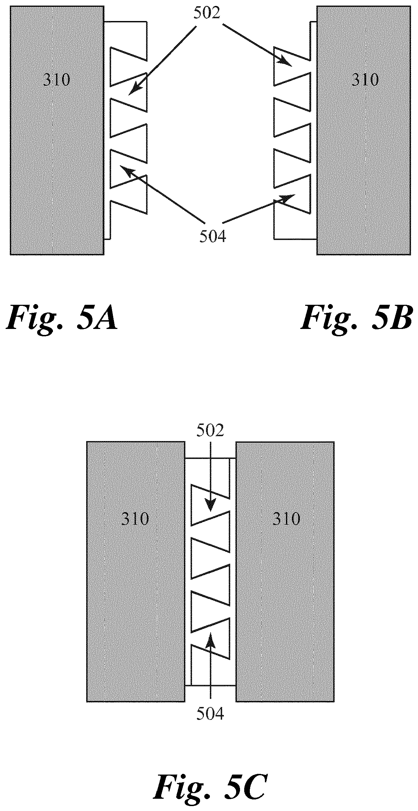

Figs. 5A and 5B depict a conjugate pair of attachment modules using a lock-and-key type attachment mechanism. The attachment modules of Fig. 5A and 5B may be the same, which is identifiable by their rotational symmetry. However, the use of the lock-and-key type attachment mechanism may also be achieved by conjugate pairs of attachment modules that are not the same. Each contact portion of the attachment modules of Fig. 5A and 5B includes both teeth (502) and voids (504). The teeth (502) and voids (504) both act in combination to both guide the connection of the attachment modules, as well as affixing the attachment modules into place.

Fig. 5C depicts the lock-and-key attachment mechanism of Figs. 5A and 5B in the post-attachment state. Such a state may be achieved by moving the first attachment module (shown in Fig. 5A), relative to the second attachment module (shown in Fig.5B) along a vector that is orthogonal to the attachment axis. For instance, in the diagram of Fig. 5, the attachment modules shown in Figs. 5A and 5B would be moved relative to one another along a vector that is orthogonal to the plane of the page in order to arrive at the post-attachment state shown in Fig. 5C.

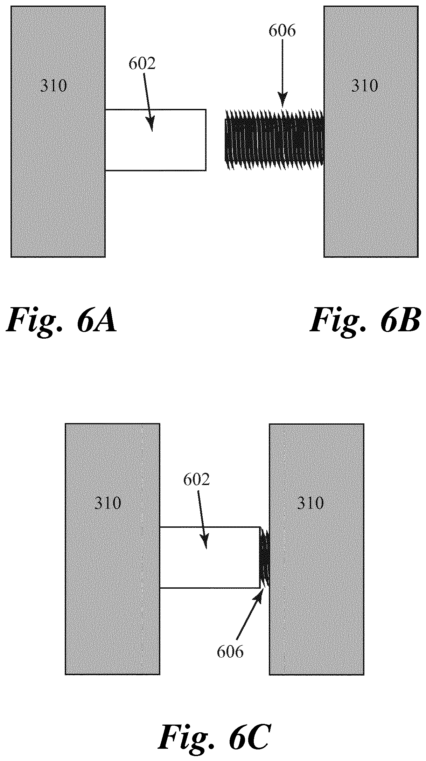

Figs. 6A and 6B depict a conjugate pair of attachment modules using a nut-and-bolt type attachment mechanism. Fig. 6A depicts an attachment module that includes a nut element (602) which is threaded on the interior to receive the bolt element (606) shown in the attachment module of Fig. 6B.

Fig. 6C depicts the nut-and-bolt attachment mechanism of Figs. 6A and 6B in the post-attachment state. Such a state may be achieved by inserting the bolt element (606) into the nut element (602), and rotating the bolt element (606) relative to the nut element (602) until the two attachment modules are affixed to one another.

The attachment modules shown in Figs. 3-6 are only examples; any suitable attachment mechanism may be used in a composite solid cleansing composition according to the present specification. If more than one interface between solid cleansing compositions is included in a composite cleansing composition according to the present specification, then the conjugate pairs of attachment modules may be the same or different. For example, one attachment module pair may use the attachment mechanism diagrammed in Figs. 3A-3C, while another attachment module pair may use the attachment mechanism diagrammed in Figs. 6A-6C. Varying the attachment mechanism may prevent a user from placing chemically incomptabible solid cleansing compositions adjacent to one another in a composite solid cleansing composition.

The attachment modules may be prepared from any suitable material. For example, the attachment modules may be plastic. In another example, the attachment modules may be metal.

The attachment modules may be either detachable or not detachable. A detachable attachment module is an attachment module that is able to interact with its conjugate attachment module, but whose interaction with the conjugate attachment module is reversible. An attachment module that is not detachable interacts with its conjugate attachment module in a manner that prevents disassembly of the composite solid cleansing composition. For example, the conjugate pairs of attachment modules shown in Figs. 4A-6C may be detachable, while the conjugate pair of attachment modules shown in Figs. 3A-3C may be not detachable.

The attachment modules themselves may also be either the same or different. An attachment module is the same as its conjugate module if two identical contact parts of the attachment modules can interact to generate the post-attachment state. An example of an attachment module being the same as its conjugate attachment module is shown in Fig. 5. An attachment module is different from its conjugate module if two identical contact parts of the attachment modules cannot interact to generate the post-attachment state. Examples of an attachment module being different from its conjugate module are shown in Figs. 3, 4 and 6.

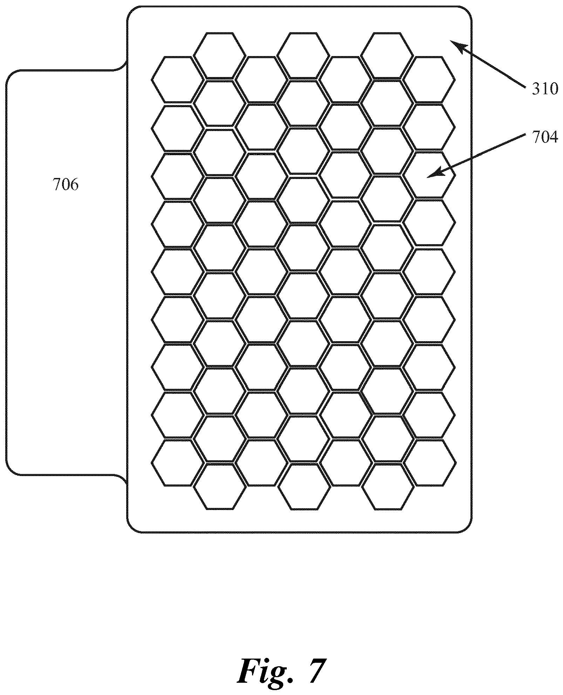

Fig. 7 depicts a part of an attachment module that anchors the attachment module into a solid cleansing composition, according to an example of the principles described herein. Fig. 7 shows an anchor part (310) of an attachment module that is equipped with holes (704). In Fig. 7, the holes (704) are shown as hexagons, and the holes (704) in combination form a hexagonal lattice. The anchor part (310) rests within the solid cleansing composition, and the solid cleansing composition may also fill any holes (704) present on the anchor part (310). In Fig. 7, the contact part (706) attachment module is shown generically, and any suitable contact part may be used. For example, one of the contact parts shown in Figs. 3A, 3B, 4A, 4B, 5A, 5B, 6A, and 6B may be used. An anchor part (310) of an attachment module may be embedded within a solid cleansing composition. In one example, positioning the anchor part (310) of an attachment module within a solid cleansing composition may be referred to as embedding the attachment module in the solid cleansing composition, and affixes the attachment module within the solid cleansing composition.

An anchor part (310) according to the present specification may also have a smooth or roughened surface. Surface roughness (R_{z}) may be measured using ISO 4287 (DIN 4728, 2009 revision). The surface roughness, R_{z}, may be the arithmetic mean of the absolute value of the profile departure with a provided length. For example, an anchor part (310) according to the present specification may have a surface that is, or is perceived as, smooth. In another example, an anchor part (310) according to the present specification may have surface that is, or is perceived as, rough. Such a rough surface may have a surface roughness, R_{z} of between 1.0 micrometer (µm) and 5.0 millimeters (mm) as measured by ISO 4287. In a further example, an anchor part (310) according to the present specification may have a surface roughness, R_{z} of between 50 µm and 2.0 mm, such as between 500 µm and 1.0 mm, as measured by ISO 4287. A roughened surface may enhance the ability of the anchor part (310) to anchor into the solid cleansing composition, both by increasing the surface area over which the anchor part (310) interacts with the solid cleansing composition, as well as by providing a grip between the anchor part (310) and the solid cleansing composition. An anchor part (310) according to the present specification may include at least one hole (704), the anchor part (310) may also be provided without holes (704).

An anchor part (310) that includes holes (704) may include any type, number, size, or shape of hole (704). For example, an anchor part (310) may include a single circular hole (704). In another example, an anchor part (310) may include a grid of circular holes (704). In a further example, an anchor part (310) may include a number of holes that are shaped and arranged so as to either replicate or create the impression of a picture, letter, logo, or the like.

In order to provide a user with the ability to select the components of a solid cleansing composition, such solid cleansing compositions may be provided with a variety of components. Such components may include a surfactant, a perfume, a moisturizing ingredient, a polymer, an antibacterial agent, a humectant, a dye, a pigment, an exfoliant, a conditioning agent, a plant extract, plant matter, an essential oil, an oil, a wax, a silicone oil, a silicone wax, a chelator, a vitamin, a vitamin derivative, an alkali metal halide, a pH-adjusting agent, an acne treatment agent, and combinations thereof. The above components may be present in differing amounts, and/or in differing compositions to allow a user to customize a composite solid cleansing composition from component solid cleansing compositions, according to the user's preferences.

A solid cleansing composition according to the present specification may include at least one surfactant. A surfactant may have a hydrophobic end and a hydrophilic end. The hydrophobic end may allow the surfactant to interact with uncharged molecules, such as oils. The hydrophilic end may facilitate the interaction of the molecule with charged or polar molecules, such as water. The hydrophilic end may be used to classify surfactants, which may be anionic, cationic, nonionic, amphoteric, or zwitterionic. Anionic surfactants may have a negatively charged hydrophilic end. Examples of anionic surfactants include sulfate, sulfonate, carboxylate, phosphate, or the like. Anionic surfactants may be sensitive to water hardness. Cationic surfactants may be those that have a positively charged hydrophilic end, such as a quaternary amine. Nonionic surfactants may have a hydrophilic end which may be charge neutral, such as an ethoxylate, glycoside, or poly-ol; such surfactants may not be sensitive to water hardness. Amphoteric surfactants may be those that have a hydrophilic end which has a functional group that is capable of acting as a base, and a functional group that is capable of acting as an acid, such as amine oxides. Zwitterionic surfactants may have both a positive and negative charge on their hydrophilic ends, such as sultaines, or betaines. The hydrophobic end may include a saturated or unsaturated, linear or branched, substituted or unsubstituted, cyclic or acyclic alkyl chain containing at least 8 carbon atoms.

For the purposes of the present specification, "alkyl" may refer to saturated or unsaturated, branched or unbranched, cyclic or acyclic, substituted or unsubstituted hydrocarbon chains of any length. For example, alkyl may refer to saturated hydrocarbon chains, such as lauryl groups (-C₁₂H₂₅), myristyl groups (-C₁₄H₂₉), cetyl groups (-C₁₆H₃₃), stearyl groups (-C₁₈H₃₇), isostearyl groups (-C₁₈H₃₇), and the like. In another example, alkyl may refer to unsaturated hydrocarbon chains, such as oleyl groups (-C₁₈H₃₅), linoleyl groups (-C₁₈H₃₃), and the like. In a further example, alkyl may refer to hydrocarbons bearing additional heteroatom substituents, such as ricinoleyl groups (-C₁₈H₃₅O), and the like. In a still further example, alkyl may refer to hydrocarbons bearing cyclic groups, which may optionally contain heteroatoms, such as dodecylbenzyl groups (-C₁₈H₂₉), dodecylpyridinyl groups (-C₁₇H₂₉N), and the like.

Anionic surfactants may include alkyl carboxylic acids, alkyl ether carboxylic acids, alkyl phosphates, alkyl ether phosphates, alkyl sulfates, alkyl ether sulfates, alkyl sulfonates, alkyl ether sulfonates, and salts thereof. In principle, any type, number, or combination of anionic surfactants may be used in solid cleansing compositions according to the present specification; selected examples are provided below.

A soap may be included as an ingredient in the solid cleansing composition. A soap is a salt of an alkyl carboxylic acid. For example, a soap may be an ammonia, alkali or alkaline earth metal salt of a fatty carboxylic acid. A soap that is an alkaline earth metal salt may be either a divalent salt of two fatty acid chains or a single fatty acid chain and another anion, for example a hydroxide ion (⁻OH).

Suitable non-limiting examples of soaps which may be used according to the present specification include sodium, potassium or lithium salts of caprylic acid, capric acid, lauric acid, myristic acid, palmitic acid, stearic acid, arachidic acid, behenic acid, isostearic acid, hydroxystearic acid, and dihydroxystearic acid.

Additional non-limiting examples of soaps which may be used according to the present specification include soaps which are prepared by saponification of triglycerides and that are not purified to homogeneity. Such soaps may include, for example, sodium, potassium and lithium soaps made from coconut oil, olive oil, rapeseed oil, castor oil, shea butter, cocoa butter, palm oil, and avocado oil.

Soaps may be included in solid cleansing compositions according to an example of the present specification in concentrations ranging from 0% to 95% by weight. For example, a soap may be included in a solid cleansing composition according to the present specification at a concentration ranging from 30% to 90%. In another example, a solid cleansing composition according to the present specification may contain a soap at a concentration ranging from 45% to 85%. In a further example, a concentration of soap ranging from 55% to 80% may be used in solid cleansing compositions according to the present specification. In a still further example, a soap concentration ranging from 65% to 75% may be used in solid cleansing compositions according to the present specification. In another example, soaps may be included in a solid cleansing composition according to the present specification in concentrations ranging from 5% to 95%. In another example, soaps may be included in a solid cleansing composition according to the present specification at concentrations ranging from 4% to 10%. All of the above concentrations are provided as percentages by weight, relative to the total weight of the composition.

Another type of anionic surfactant which may be used according to an example of the present specification is an alkyl carboxylic acid type of surfactant. Alkyl carboxylic acids are fatty acids which are provided in the protonated (acid) form. Suitable non-limiting examples of alkyl carboxylic acids are the soaps noted above when provided in the acid form.

Another type of anionic surfactant which may be used according to an example of the present specification include alkyl sulfates. Alkyl sulfates may also be used according to the present specification as salts of ammonia, alkali or alkaline earth metals. Suitable non-limiting examples of alkyl sulfates which may be used according to an example of the present specification include lauryl sulfate, myristyl sulfate, cetyl sulfate, stearyl sulfate, behenyl sulfate, and salts and/or mixtures thereof. For example, sodium, potassium or ammonium salts of the above alkyl sulfates may be used.

Another type of anionic surfactant which may be used according to an example of the present specification include an alkyl ether sulfate type of surfactant, which are surfactants that contain an ether linkage separating the alkyl group from the sulfate group. In principle, any ether linkage of a diol or polyol may be used. For example, ethylene or polyethylene glycol ether linkages may be used. In another example, propylene or polypropylene glycol ethers may be used. In a further example, glyceryl or polyglyceryl ether linkages may be used. In a still further example, butylene or polybutylene glycol ethers may be used. Alkyl ether sulfates may be provided in either the protonated form or as salts of ammonia, alkali or alkaline earth metals. Suitable non-limiting examples of alkyl ether sulfates which may be used according to an example of the present specification include laureth-2 sulfate, laureth-3 sulfate, laureth-5 sulfate, laureth-6 sulfate, laureth-10 sulfate, laureth-12 sulfate, myreth-2 sulfate, myreth-5 sulfate, myreth-10 sulfate, and mixtures and/or salts thereof.

Another type of anionic surfactant which may be used according to an example of the present specification is an alkyl ether carboxylic acid type of surfactant. Alkyl ether carboxylic acids may be surface-active agents which have a carboxylic group as the hydrophilic group, and an ether linkage between the carboxylic acid group and the alkyl chain. In principle, any ether linkage of a diol or polyol may be used. For example, ethylene or polyethylene glycol ether linkages may be used. In another example, propylene or polypropylene glycol ethers may be used. In a further example, glyceryl or polyglyceryl ether linkages may be used. In a still further example, butylene or polybutylene glycol ethers may be used. Alkyl ether carboxylic acids may be provided in either the protonated (acid) form, or as salts of ammonia, alkali or alkaline earth metals. Suitable non-limiting examples of alkyl ether carboxylic acids include butoxynol-5 carboxylic acid, ceteareth-13 carboxylic acid, ceteareth-25 carboxylic acid, coceth-7 carboxylic acid, laureth-3 carboxylic acid, laureth-4 carboxylic acid, laureth-5 carboxylic acid, laureth-6 carboxylic acid, laureth-8 carboxylic acid, laureth-10 carboxylic acid, myreth-2 carboxylic acid, myreth-3 carboxylic acid, myreth-5 carboxylic acid, isosteareth-6 carboxylic acid, isosteareth-11 carboxylic acid, oleth-3 carboxylic acid, oleth-6 carboxylic acid, oleth-10 carboxylic acid, and mixtures and/or salts thereof.

Another type of anionic surfactant which may be used according to an example of the present specification is an alkyl sulfonate type of surfactant. Alkyl sulfonates are surfactants that have an alkyl group directly linked to the sulfur of the sulfonate group. Alkyl sulfonates may also be provided as salts of ammonia, alkali or alkaline earth metals. Suitable non-limiting examples of alkyl sulfonate type surfactants which may be used in accordance with an example of the present specification include sodium cocoyl isethionate, sodium lauroyl isethionate, taurate, cetearyl sulfosuccinate, C13-17 alkane sulfonate, C14-18 alkane sulfonate, cocoamphohydroxypropylsulfonate, C12-14 olefin sulfonate, C14-16 olefin sulfonate, C16-18 olefin sulfonate, and mixtures and/or salts thereof.

Another type of anionic surfactant which may be used according to an example of the present specification is an alkyl phosphate type of surfactant. Alkyl phosphates are surfactants that contain a phosphate group as the hydrophilic group, and contain at least one alkyl group. Alkyl phosphates may be provided in either the protonated form or as an ammonia, alkali or alkaline earth metal salt. Suitable non-limiting examples of alkyl phosphate surfactants which may be used in accordance with an example of the present specification include phospholipid SV (stearamidopropyl PG-dimonium chloride phosphate), lauryl phosphate, dilauryl phosphate, myristyl phosphate, dimyristyl phosphate, cetyl phosphate, dicetyl phosphate, stearyl phosphate, distearyl phosphate, behenyl phosphate, dibehenyl phosphate, oleyl phosphate, and salts and/or mixtures thereof.

Another type of anionic surfactant which may be used according to an example of the present specification is an alkyl ether phosphate type of surfactant. Alkyl ether phosphates may be surface-active agents bearing an ether linkage between at least one alkyl group and the phosphate group. In principle, any ether linkage of a diol or polyol may be used. For example, ethylene or polyethylene glycol ether linkages may be used. In another example, propylene or polypropylene glycol ethers may be used. In a further example, glyceryl or polyglyceryl ether linkages may be used. In a still further example, butylene or polybutylene glycol ethers may be used. Alkyl ether phosphates may be provided in either their protonated form or as an ammonia, alkali or alkaline earth metal salt. Suitable non-limiting examples of alkyl ether phosphates include oleth-3 phosphate, oleth-10 phosphate, and mixtures and/or salts thereof.

Anionic surfactants may be included in solid cleansing compositions according to an example of the present specification separately from soaps, and may be included in amounts ranging from 0% to 50% by weight. For example, concentrations of the anionic surfactants other than soaps may range from 1% to 20%. Still further concentrations of the anionic surfactants other than soaps may range from 1.5% to 10%. All concentrations are provided as a percentage by weight, relative to the total weight of the composition.

Solid cleansing compositions according to an example of the present specification may also contain nonionic surfactants. For example, the solid cleansing composition may include alkoxylated fatty alcohols, alkoxylated fatty esters, alkanolamides, alkyl glycosides, and combinations thereof.

Alkoxylated fatty alcohols may be incorporated in solid cleansing compositions according to the present specification as a nonionic surfactant. Alkoxylated fatty alcohols are condensation products of a number of alkoxy groups with a fatty alcohol. In principle, any type or number of alkoxy groups may be used. For example, ethylene glycol or polyethylene glycol, propylene or polypropylene glycol, glyceryl or polyglyceryl, butylene or polybutylene glycol may be used. In a further example, combinations of the above may be used, such as a combination of polyethylene glycol and polypropylene glycol, as either a random- or block-condensation product. Suitable non-limiting examples of alkoxylated fatty alcohols for use in solid cleansing compositions according to the present specification are ceteth-2, ceteth-10, ceteth-20, ceteth-25, ceteareth-10, ceteareth-12, ceteareth-20, ceteareth-25, steareth-2, steareth-10, steareth-20, laureth-10, PPG-4-ceteth-20, and combinations thereof.

Another type of nonionic surfactant which may be used in solid cleansing compositions according to an example of the present specification is an alkoxylated fatty ester type of surfactant. Alkoxylated fatty esters are esters of fatty acids with at least one alkoxy group. In principle, any type or number of alkoxy groups may be used. For example, ethylene glycol or polyethylene glycol, propylene or polypropylene glycol, glyceryl or polyglyceryl, butylene or polybutylene glycol may be used. In a further example, combinations of the above may be used, such as a combination of polypropylene glycol and polyethylene glycol. Suitable non-limiting examples of alkoxylated fatty esters include glyceryl stearate, PEG-2-PPG-5 laurate, PEG-2 stearate, PEG-20 stearate, PEG-100 stearate, and the like.

Another type of nonionic surfactant which may be used in solid cleansing compositions according to the present specification is an alkanolamide type of surfactant. Suitable non-limiting examples of alkanolamides which may be used in solid cleansing compositions according to the present specification include coco monoethanolamide (MEA), lauric MEA, lauric DEA, myristic MEA, stearic MEA, stearic DEA, behenic MEA, and combinations thereof.

Another type of nonionic surfactant which may be used in solid cleansing compositions according to the present specification is an alkyl glycoside type of surfactant. Alkyl glycosides are condensation products of fatty alcohols with a number of sugars. While each molecule of an alkyl glycoside contains an integer number of sugars, the ensemble average may be a noninteger number. For example, an alkyl glycoside surfactant could contain glucose as the sugar, and could be included as a mixture of compounds with 2, 3, 4 and 5 glucose units, such that the ensemble average contains 3.4 glucose units. Suitable non-limiting examples of alkyl glycosides which may be used in solid cleansing compositions according to an example of the present specification include coco glucoside and lauryl glucoside.

Nonionic surfactants may be incorporated into solid cleansing compositions according to an example of the present specification at concentrations ranging from 0% to 50% by weight. For example, a solid cleansing composition according to the present specification may contain at least one nonionic surfactant at a concentration ranging from 0.01% to 15% by weight. In another example, a solid cleansing composition according to the present specification may contain a nonionic surfactant at a concentration ranging from 0.1% to 10% by weight. In a further example, a solid cleansing composition according to the present specification may contain a nonionic surfactant at a concentration ranging from 0.1% to 5% by weight. All of the above concentrations are provided as weight percentages, relative to the total weight of the composition.

Solid cleansing compositions according to the present specification may also include cationic surfactants. Cationic surfactants include any surfactant that contains a positive charge and does not contain a negative charge, such as, for example, quaternary ammonium salt surfactants and tertiary ammonia surfactants (which may form a quaternary ammonium surfactant in compositions with a pH of less than about 9). Additional types of cationic surfactants which may be used in solid cleansing compositions according to the present specification include esterquat and amidoamine surfactants.

Suitable non-limiting examples of cationic surfactants which may be used in solid cleansing compositions according to the present specification include cetrimonium chloride, cetrimonium methosulfate, steartrimonium chloride, steartrimonium methosulfate, behentrimonium chloride, behentrimonium methosulfate, stearamidopropyl trimonium methosulfate, behenamidopropyl trimonium methosulfate, stearamidopropyl dimethylamine, palmitamidopropyl dimethylamine, and the like.

Cationic surfactants may be incorporated in solid cleansing compositions according to an example of the present specification in concentrations ranging from 0% to 50% by weight, for example from 0.01% to 15% by weight. In another example, cationic surfactants may be included at concentrations ranging from 0.1% to 10% by weight. In a further example, cationic surfactants may be included in solid cleansing compositions according to the present specification at concentrations ranging from 1% to 5% by weight. A still further example may contain cationic surfactants in a solid cleansing composition according to the present specification in the range of 0.1% to 2% by weight, with all weights being relative to the total weight of the composition.

Solid cleansing compositions according to examples the present specification may also include amphoteric surfactants, which are surfactants which have a hydrophilic part which has both acidic and basic hydrophilic groups and which behaves in an acidic or basic manner, depending on the conditions. Unlike zwitterionic surfactants, amphoteric surfactants do not permanently bear a charge. Amphoteric surfactants include surfactants based on aliphatic amines having carboxy, sulfo or phosphono side chains. Amphoteric surfactants also include such surfactants as N-alkyl glycines, N-alkyl propionic acids, N-alkyl aminobutyric acids, N-alkyl taurines, N-alkyl sarcosines, and amine oxide surfactants.

Suitable non-limiting examples of amphoteric surfactants which may be included in solid cleansing compositions according to the present specification include lauryldimethylamine oxide, disodium cocoamphodiacetate, disodium lauroamphodiacetate, sodium lauroamphoacetate, disodium cocoamphodipropionate, and combinations thereof.

Amphoteric surfactants may be included in solid cleansing compositions according to an example of the present specification at concentrations ranging from 0% to 50% by weight. For example, a solid cleansing composition according to the present specification may contain an amphoteric surfactant at a concentration ranging from 0.01% to 25% by weight. In another example, a solid cleansing composition according to the present specification may contain an amphoteric surfactant at a concentration ranging from 0.1% to 15% by weight. In a further example, at least one amphoteric surfactant may be present in a solid cleansing composition according to the present specification at a concentration ranging from 1% to 10% by weight. In a still further example, an amphoteric surfactant may be present in a solid cleansing composition according to the present specification at a concentration ranging from 0.1% to 5% by weight, with all weights being relative to the total weight of the composition.

Solid cleansing compositions according to the present specification may also include zwitterionic surfactants. Zwitterionic surfactants are surfactants which bear both a positive charge and a negative charge. Some types of zwitterionic surfactants are capable of forming intramolecular salts. Zwitterionic surfactants include the betaine type of surfactants as well as the sultaine type of surfactants.

Suitable non-limiting examples of zwitterionic surfactants which may be included in solid cleansing compositions according to the present specification include cocamidopropyl betaine, cocamidopropyl sultaine, cocamidopropyl hydroxysultaine, laurylamidopropyl betaine, laurylamidopropyl sultaine, stearylamidopropyl betaine, oleyl betaine, myristyl betaine, stearyl betaine, cetyl betaine, and the like. Combinations of the above are also suitable for incorporation into solid cleansing compositions according to the present specification.

Zwitterionic surfactants may be included in solid cleansing compositions according to an example of the present specification at concentrations ranging from 0% to 50% by weight. For example, a solid cleansing composition according to the present specification may contain a zwitterionic surfactant at a concentration ranging from 0.01% to 25% by weight. In another example, a solid cleansing composition according to the present specification may contain a zwitterionic surfactant at a concentration ranging from 0.1% to 15% by weight. In a further example, at least one zwitterionic surfactant may be present in a solid cleansing composition according to the present specification at a concentration ranging from 1% to 10% by weight. In a still further example, a zwitterionic surfactant may be present in a solid cleansing composition according to the present specification at a concentration ranging from 0.1% to 5% by weight, with all weights being relative to the total weight of the composition.

A solid cleansing composition according to the present specification may also include a perfume. A perfume is a component or composition which produces an olfactory sensation in an individual. A perfume may contain a single component; a perfume may also be a mixture of multiple separate components. Non-limiting examples of perfume components which may be used in solid cleansing compositions according to the present specification include aldehydes, ketones, aromatic hydrocarbons, aromatic alcohols and combinations thereof. Non-limiting examples of suitable perfume ingredients include alpha-hexyl cinnamal, vanillin, citral, eugenol, geraniol, limonene, and citronellol. A solid cleansing composition according to the present specification may contain any suitable amount of a perfume, such as up to 5% by weight, relative to the total weight of the composition. For example, a solid cleansing composition according to the present specification may contain from 0.1% to 2.0% by weight of a perfume, relative to the total weight of the composition.

A solid cleansing composition according to the present specification may also include a moisturizing ingredient. Moisturizing ingredients are chemical agents that cause the skin to be softer and to have a higher moisture content. Moisturizing ingredients may act by reducing evaporation from the surface of the skin, or by attracting water to the surface of the skin. Moisturizing ingredients include oils, silicone oils, waxes, silicone waxes, humectants, conditioning agents, and any other agent that acts to increase the moisture content of the skin or decrease the amount of oil that is removed from the skin by a surfactant in the solid cleansing composition. Non-limiting examples of specific moisturizing ingredients which may be suitable for use in a solid cleansing composition according to the present specification include shea butter, castor oil, cetearyl palmitate, propylene glycol, and combinations thereof. A solid cleansing composition according to the present specification may contain any suitable amount of a moisturizing ingredient, such as up to 25% by weight, relative to the total weight of the composition. For example, a solid cleansing composition according to the present specification may contain from 5% to 15% of a moisturizing ingredient.

A solid cleansing composition according to the present specification may also contain a polymer. Polymers may be natural, synthetic, or semisynthetic. A natural polymer is a polymer that is assembled enzymatically as a natural result of biological processes. A synthetic polymer is a polymer that is assembled from monomeric units by synthetic processes. Semi-synthetic polymers include chemically modified natural polymers, polymers assembled from both natural and synthetic monomers, and the like. A polymer may be included in a solid cleansing composition according to the present specification as a solidifier or thickener, a conditioning agent, an exfoliant, or to confer other rheological properties to the solid cleansing composition. Non-limiting examples of suitable polymers include hydrolyzed keratin, silk, honey, collagen, cellulose, chemically modified collagen, chemically modified cellulose, and acrylates copolymers. A polymer may be incorporated into a solid cleansing composition according to the present specification in any suitable amount, such as up to 10% by weight, relative to the total weight of the composition. For example, a solid cleansing composition according to the present specification may contain from 0.1% to 5% by weight of a polymer, relative to the total weight of the composition.

A solid cleansing composition according to the present specification may also contain an antibacterial agent. An antibacterial agent may be any agent which assists in the removal of bacteria, kills bacteria, or arrests bacterial growth. Suitable non-limiting examples of antibacterial agents include antiseptics, triclosan, benzethonium salts, benzalkonium salts, compounds which inhibit the 70S (bacterial) ribosome, compounds which reduce the integrity of the bacterial cell wall, and compounds which sequester nutrients-for example, metal ions-that bacteria require. Additional non-limiting examples of antibacterial agents include aminoglycosides (such as neomycin), cephalosporins (such as cefalexin), lincosamides (such as lincomycin), tetracyclines (such as doxycycline), penicillins (such as amoxicillin), chelating agents (such as ethylenediaminetetraacetic acid), and combinations thereof. A solid cleansing composition according to the present specification may contain any suitable amount of an antibacterial agent, such as up to 10% by weight, relative to the total weight of the composition. For example, a solid cleansing composition according to the present specification may contain from 0.1% to 5% by weight of an antibacterial agent, relative to the total weight of the composition.

A solid cleansing composition according to the present specification may also contain a humectant. Humectants are hygroscopic substances, or substances which attract water. Humectants may be added to an example of solid cleansing compositions according to the present specification in order to maintain moisture on the skin to which the solid cleansing composition is applied. Non-limiting examples of humectants which may be used in solid cleansing compositions according to the present specification may include polyols, urea, honey, aloe vera gels, glycerol, sorbitol, glycols, propylene glycol, and butylene glycol. A solid cleansing composition according to the present specification may contain any suitable amount of a humectant, such as up to 10% by weight, relative to the total weight of the composition. For example, a solid cleansing composition according to the present specification may contain from 0.1% to 5% by weight of a humectant, relative to the total weight of the composition.

A solid cleansing composition according to the present specification may also contain a dye or pigment. Dyes and pigments are compounds which confer color to a solid cleansing composition. Dyes and pigments may be added to an example of a solid cleansing composition according to the present specification in order to imbue the solid cleansing composition with a consumer-acceptable color. Non-limiting examples of dyes and pigments which may be used to color the solid cleansing composition according to the present specification include titanium dioxide, mica, iron oxides, violet 2, red 4, red 6, red 7, red 33, red 40, blue 1, blue 4, yellow 5, yellow 6, yellow 10, orange 4, orange 5, orange 10, vat red 1, vat blue 1, vat blue 4, vat blue 6, vat orange 7, vat violet 2, and combinations thereof. A solid cleansing composition according to the present specification may include any suitable quantity of a dye and/or pigment, such as up to 5% by weight, relative to the total weight of the composition. According to one example, dyes and/or pigments may be used in distinct solid cleansing compositions to allow a user to readily identify that a composite solid cleansing composition is a mixture of distinct solid cleansing compositions, as well as allowing a user to identify by color the specific adjuvants or benefit agents that are present in a solid cleansing composition according to the present specification.

A solid cleansing composition according to the present specification may also contain an exfoliant. Exfoliants are particles which are non-dissolvable solids which may be dispersed throughout an example of a solid cleansing composition according to the present specification in order to act as an abrasive when the solid cleansing composition is used to cleanse the skin. The abrasive properties of the exfoliating particles may act to remove dead skin cells from the surface of the skin in order to allow deeper action of a surfactant and/or a moisturizing ingredient, and promote regeneration of the skin. Suitable non-limiting examples of exfoliants which may be used in solid cleansing compositions according to the present specification include *actinidia chinensis* (kiwi) seed, attapulgite, *avena sativa* (oat) bran, chalk, *cocos nucifera* (coconut) shell powder, diatomaceous earth, *helianthus annuus* (sunflower) seed meal, *juglans mandshurica* (walnut) shell powder, *olea europaea* (olive) husk powder, silica, talc, volcanic ash, wood powder, or combinations thereof. A solid cleansing composition according to the present specification may include any suitable amount of an exfoliant, such as up to 5% by weight, relative to the total weight of the composition.

A solid cleansing composition according to the present specification may also contain a conditioning agent. Conditioning agents act to preserve existing moisture by creating a hydrophobic barrier between the moisturized skin and the air, and may be incorporated into an example of solid cleansing compositions according to the present specification for this purpose. Non-limiting examples of conditioning agents include the polyquaternium class of polymers, fatty alcohols and polyols. A solid cleansing composition according to the present specification may include any suitable amount of a conditioning agent, such as up to 10% by weight, relative to the total weight of the composition.

A solid cleansing composition according to the present specification may also contain a plant extract. Plant extracts may be natural compounds or mixtures of compounds produced in a plant which contain a number of agents that have either a real or perceived benefit to the skin, or to the solid cleansing composition as a whole. The inclusion of some plant extracts may improve consumer acceptance of a solid cleansing composition on the basis of these benefits, or accommodate a consumer preference for naturally produced solid cleansing compositions over synthetically produced solid cleansing compositions. Plant extracts may include oils, perfume ingredients, fatty acids, and/or various other components depending on the extraction methods employed and any subsequent processing that is performed. Non-limiting plant extracts which may be used in solid cleansing compositions according to the present specification may include *Ocimum basilicum* extract, *Calendula officinalis* extract, *Matricaria chamomilla* extract, *Oenothera biennis* extract, *Zingiber officinale* extract, *Jasminum* extracts, *Lavandula angustifolia* extract, *Mentha* x *piperita* extract, *Rosmarinus officinalis* extract, *Rosa* extracts, *Hypericum perforatum* extract, *Syringa vulgaris* extract, and combinations thereof. A solid cleansing composition according to the present specification may include any suitable amount of a plant extract, such as up to 5% by weight, relative to the total weight of the composition.

A solid cleansing composition according to the present specification may also contain plant matter. Plant matter is plant material which may be incorporated into an example of solid cleansing compositions according to the present specification. Such plant material may provide abrasive properties as exfoliants, perfume properties, or as a thickener. The incorporation of plant material into solid cleansing compositions may improve consumer acceptance, which may be based on the perception of the natural qualities of the solid cleansing compositions including plant matter. Non-limiting examples of plant matter which may be incorporated in solid cleansing compositions according to the present specification include whole flowers, flower petals, stems, seeds, roots, and fruits. Non-limiting examples of plant sources which may provide the plant matter include *Citrus* plants, *Malus domestica* plants, *Hypericum perforatum* plants, and *Zingiber officinale* plants. A solid cleansing composition according to the present specification may contain any suitable amount of plant matter, such as up to 10% by weight, relative to the total weight of the composition.

A solid cleansing composition according to the present specification may also contain an essential oil. An essential oil is a particular type of plant extract, which may include volatile aroma compounds from the plant from which the essential oil is extracted. The extraction methods used may determine the composition of the essential oil. Possible extraction methods may include steam distillation, pressure, solvent extraction with organic solvents, solvent extraction with carbon dioxide, and oil extractions, for example. In principle, any type of plant may be used to prepare an essential oil, such as *Ocimum basilicum* essential oil, *Calendula officinalis* essential oil, *Matricaria chamomilla* essential oil, *Oenothera biennis* essential oil, *Zingiber officinale* essential oil, *Jasminum* essential oils, *Lavandula angustifolia* essential oil, *Mentha* x *piperita* essential oil, *Rosmarinus officinalis* essential oil, *Rosa* essential oils, *Hypericum perforatum* essential oil, and *Syringa vulgaris* essential oil. A solid cleansing composition according to the present specification may include any suitable amount of an essential oil, such as up to 5% by weight, relative to the total weight of the composition.

A solid cleansing composition according to the present specification may also contain an oil. Oils are neutral, nonpolar substances that are viscous liquids at standard ambient temperature and pressure (1 atmosphere pressure, 25° Celsius). Oils may be included in an example of solid cleansing compositions according to the present specification in order to act as conditioning agents, or to replenish natural oils on the skin. Oils may include triglycerides, fatty alcohols, and mineral oils. Mineral oils are oils that are prepared by distillation from crude oil. Non-limiting examples of oils which may be suitable for use in solid cleansing compositions according to the present specification include olive oil, vegetable oil, rapeseed oil, paraffinum liquidum, cetyl alcohol, stearyl alcohol, myristyl alcohol, octyldodecanol, oleyl alcohol, sunflower oil, corn oil, palm oil, soybean oil, sunflower oil, safflower oil, peanut oil, and combinations thereof. A solid cleansing composition according to the present specification may contain any suitable amount of an oil, such as up to 10% by weight, relative to the total weight of the composition.

A solid cleansing composition according to the present specification may also contain a wax. Waxes are compounds which have a large fatty content, and are solid at standard ambient temperature and pressure. Waxes may be malleable at standard ambient temperature and pressure. Waxes may have a melting point at or above about 45° Celsius (C). A wax may be included in an example of a solid cleansing composition according to the present specification in order to improve rheological properties, act as a conditioning agent, or to provide other such properties to the solid cleansing composition. Waxes include fatty esters, fatty ethers, hydrocarbons, primary alcohols, secondary alcohols, ketones and aldehydes. Waxes may be alkanes, alkenes, or alkynes, and may be aromatic, anti-aromatic or aliphatic. Waxes may be derived from plants, animals, or crude oil. Non-limiting examples of waxes which may be used in solid cleansing compositions according to the present specification include cetyl palmitate, lanolin, myricyl palmitate, Carnauba wax, candelilla wax, beeswax, montan wax, paraffin wax, and combinations thereof. A solid cleansing composition according to the present specification may contain any suitable amount of a wax, such as up to 15% by weight, relative to the total weight of the composition.

A solid cleansing composition according to the present specification may also contain a silicone oil. Silicone oils are neutral compounds that are liquids at standard temperature and pressure. Silicone oils are a type of oil, and may be included in an example of a solid cleansing composition according to the present specification for the same reasons, and in the same amounts noted above for oils. Silicone oils may be saturated with hydrocarbon components along a siloxy backbone, corresponding to the Si-(O-Si)ₙ chain. Silicone oils may also have functional groups incorporated therein. Such functional groups may include amines and alcohols. Non-limiting examples of silicone oils which may be incorporated in a solid cleansing composition according to the present specification include dimethicone, cyclomethicone, dimethiconol, PEG-12 dimethicone, PEG-8 dimethicone, amodimethicone, alkyl methicones, and combinations thereof.

A solid cleansing composition according to the present specification may also contain a silicone wax. Silicone waxes are silicone compounds which are solid at standard ambient temperature and pressure. Silicone waxes are a type of waxes, and may be included in an example of solid cleansing compositions according to the present specification for the same reasons, and in the same amounts noted above for waxes. Silicone oils may have a melting point at or above about 45° C. Non-limiting examples of silicone waxes which may be used in solid cleansing compositions according to the present specification include alkyl methicones, Silwax A-08, Silwax C, Silwax D-02, Silwax F, Silwax S, and combinations thereof.

A solid cleansing composition according to the present specification may also contain a chelator. Chelators are compounds which coordinate metal ions. Chelators may be included in an example of a solid cleansing composition according to the present specification as antibacterial agents, preservatives, pH regulators, or to provide other such properties to the solid cleansing composition. Non-limiting examples of chelators which may be used in solid cleansing compositions according to the present specification include natural polyacids (such as citric acid), phosphate salts (such as disodium pyrophosphate), bisphosphonates (such as etidronic acid), aminocarboxylic acids (such as ethylenediaminetetraacetic acid (EDTA), diethylenetriaminepentaacetic acid (DPTA), ethylenediamine-N,N'-disuccinic acid (EDDS), ethylenediamine-N,N'-diglutaric acid (EDDG), glycinamide-N,N'-disuccinic acid (GADS), and ethylenediamine-N,N'-bis(ortho-hydroxyphenyl acetic acid) (EDDHA)), and combinations and/or salts thereof. A solid cleansing composition according to the present specification may contain any suitable quantity of chelators, such as up to 3% by weight, relative to the total weight of the composition.

A solid cleansing composition according to the present specification may also contain a vitamin or vitamin derivative. A vitamin is an organic compound which an organism may require in limited quantities, and which the organism that may require the vitamin cannot synthesize from other precursors. Vitamins, or vitamin derivatives, may be included in an example of solid cleansing compositions according to the present specification as conditioning agents, preservatives, antioxidants, or to improve consumer acceptance of the solid cleansing composition. Non-limiting examples of vitamins which may be used in solid cleansing compositions according to the present specification include vitamin A (retinol), vitamin B₆ (pyroxidine), vitamin B₇ (biotin), vitamin B₁₂ (cyanocobalamin), vitamin C (ascorbic acid), vitamin E (tocopherols), and vitamin K (phylloquinone). For the purposes of the present specification, "vitamin" also includes derivatives and stereoisomers of vitamins, such as polyoxypropylene (2) polyoxyethylene (5) tocopherol ether, and isoascorbic acid. A solid cleansing composition according to the present specification may contain any suitable quantity of vitamins, such as up to 10% by weight, relative to the total weight of the composition.

A solid cleansing composition according to the present specification may also contain an alkali metal halide. Alkali metal halides are salts of alkali metals and halogen atoms. Alkali metal halides may be included in an example of solid cleansing compositions according to the present specification as thickeners, ionic strength modulators, or to confer other such properties to the solid cleansing composition. Non-limiting examples of alkali metal halides which may be used in solid cleansing compositions according to the present specification include lithium fluoride, lithium chloride, lithium bromide, lithium iodide, sodium fluoride, sodium chloride, sodium bromide, sodium iodide, potassium fluoride, potassium chloride, potassium bromide, potassium iodide, and combinations thereof. A solid cleansing composition according to the present specification may contain any suitable quantity of alkali metal halides, such as up to 5% by weight, relative to the total weight of the composition.

A solid cleansing composition according to the present specification may also include a pH-adjusting agent. A pH-adjusting agent may be included in a solid cleansing composition according to the present specification in order to modulate the pH of the wetted composition that comes in contact with the skin when the solid cleansing composition is contacted with wet skin. Non-limiting examples of pH-adjusting agents which may be incorporated into an example of a solid cleansing composition according to the present specification include hydroxide salts, carbonate salts, bicarbonate salts, citric acid, lactic acid, malic acid, and combinations thereof. A solid cleansing composition according to the present specification may include any suitable amount of a pH-adjusting agent, in order to achieve the desired pH of the wetted composition that comes in contact with the skin.

A solid cleansing composition according to the present specification may also include an acne treatment agent. Acne treatment agents include keratolytic skin peeling ingredients such as salicylic acid. A solid cleansing composition according to the present specification may contain any suitable amount of an acne treatment agent, such as up to 5% by weight, relative to the total weight of the composition.

The above amounts of each component in a solid cleansing composition according to the present specification relate to the amount that is present in a single solid cleansing composition, which is then incorporated into a composite cleansing composition.

### Methods

The present specification also includes a method of making a composite solid cleansing composition (800) from component solid cleansing compositions. The method (800) includes providing (801) at least two solid cleansing compositions, each of which includes an attachment module. The method further comprises attaching (802) the at least two solid cleansing compositions through their attachment modules.

In one example, the method (800) of making a composite solid cleansing composition from component solid cleansing compositions may be accomplished by application of a force along the attachment axis. Such an attachment may be achieved using attachment mechanisms like those diagramed, for example, in Figs. 3A-3C, 4A-4C, and 6A-6C. In another example, the method (800) of making a composite solid cleansing composition from component solid cleansing compositions may be accomplished by application of a force orthogonal to the attachment axis. Such an attachment may be achieved using attachment mechanisms like those diagramed, for example, in Figs. 5A-5C.

The present specification also includes a method (900) of making a solid cleansing composition for incorporation into a composite solid cleansing composition. The method comprises providing (901) an attachment module; the attachment module includes a first part and a second part. The first part of the attachment module includes a contact point to contact and interface with a contact point of another attachment module. The second part of the attachment module includes an anchor part (310) to anchor the attachment module in the cleansing composition. The method further comprises forming (902) a solid cleansing composition around the second part of the attachment module.

The method (900) of making a solid cleansing composition for incorporation into a composite solid cleansing composition may further involve placing the attachment module into a mold and pouring a molten cleansing composition into the mold so that the cleansing composition hardens around the second part of the attachment module. The molten cleansing composition may fill holes that may be included in the second part of the attachment module.

Alternatively, the method (900) of making a solid cleansing composition for incorporation into a composite cleansing composition may further involve preparation of the solid cleansing composition, partial or complete insertion of the attachment module into the solid cleansing composition, and pressing of the solid cleansing composition to compress the solid cleansing module around the second part of the attachment module that includes an anchor part (310). If the anchor part (310) includes holes (704), then the pressing operation may fill the holes (704) with the solid cleansing composition.

### Examples

The examples that follow indicate composite solid cleansing compositions that may be prepared in accordance with the present specification. Unless otherwise indicated, the stated quantities are percentages by weight.

Example 1: Two-part composite solid cleansing composition with perfume combination.

**Table (I)**

| **Component** | **Composition 1** | **Composition 2** |
|---|---|---|
| Sodium Soap(s) | 65-91 | 65-91 |
| Additional Surfactant(s) | 0-5 | 0-5 |
| Fatty Acid(s) | 0-7 | 0-7 |
| Chelating Agent(s) | 0.01-1 | 0.01-1 |
| Alkali Metal Halide(s) | 0-2 | 0-2 |
| Humectant(s) | 1-5 | 1-5 |
| Glycerin | 3-20 | 3-20 |
| Perfume #1 | 0.5-2 | - |
| Perfume #2 | - | 0.5-2 |
| Colorant(s) | 0-0.5 | 0-0.5 |
| Water | 5-20 | 5-20 |

In example 1, a two-part composite solid cleansing composition is provided. Each part uses a distinct perfume accord, and the perfumes may be combined in a manner in accordance to a user's preferences. Other than the perfume accord, the base composition of composition 1 and composition 2 of the composite solid cleansing composition may be the same.

Example 2: Three-part composite solid cleansing composition with distinct additives.

**Table (II)**

| **Component** | **Composition 3** | **Composition 4** | **Composition 5** |
|---|---|---|---|
| Sodium Soap(s) | 55-81 | 65-91 | 65-91 |
| Additional Surfactant(s) | 5-20 | 0-5 | 0-5 |
| Chelating Agent(s) | 0.01-1 | 0.01-1 | 0.01-1 |
| Fatty Acid(s) | 0-7 | 0-7 | 0-7 |
| Alkali Metal Halide(s) | 0-2 | 0-2 | 0-2 |
| Humectant(s) | 1-5 | 1-5 | 1-5 |
| Glycerin | 3-20 | 3-20 | 3-20 |
| Moisturizer(s) | 0.5-5 | - | - |
| Perfume | - | 0.1-2 | - |
| Exfoliant(s) | - | - | 1-5 |
| Colorant(s) | 0-0.5 | 0-0.5 | 0-0.5 |
| Water | 5-20 | 5-20 | 5-20 |

In example 2, a three-part composite solid cleansing composition is provided. Each part uses a distinct additive: composition 3 includes a moisturizer; composition 4 includes a perfume; composition 5 includes an exfoliant. The base soap composition according to example 2 may be either the same or different for compositions 3, 4 and 5.

Example 3: Two-part composite soap-free solid cleansing composition.

**Table (III)**

| **Component** | **Composition 6** | **Composition 7** |
|---|---|---|
| Amphoteric Surfactant(s) | 20-50 | 20-50 |
| Cationic Surfactant(s) | 0-30 | 10-30 |
| Additional Surfactant(s) | 1-30 | - |
| Fatty Acid(s) | 0-7 | 0-7 |
| Chelating Agent(s) | 0.01-1 | 0.01-1 |
| Alkali Metal Halide(s) | 0-2 | 0-2 |
| Humectant(s) | 1-5 | 1-5 |
| Perfume | 0.5-2 | 0.5-2 |
| Wax(es) | - | 1-5 |
| Moisturizer(s) | 0.5-5 | - |
| Essential Oil | - | 0.01-2 |
| Colorant(s) | 0-0.5 | 0-0.5 |
| Water | 5-20 | 5-20 |

In example 3, a two-part solid cleansing composition is provided that does not contain soaps. Such compositions may be appealing to consumers based on the impression that a cleansing composition that does not include soaps will be less drying to a user's skin, and may be referred to as synthetic detergent ("syndet") compositions. Composition 6 and composition 7 may include several different components, and provide that a user may be able to select compositions that differ by a number of elements. For example, composition 6 contains additional surfactants and moisturizers that are not present in composition 7, while composition 7 contains waxes and an essential oil that are not present in composition 6. Compositions 6 and 7 may have either the same or a different base composition.

## Claims

1. A composite solid cleansing composition, comprising:
at least two solid cleansing compositions (102, 104, 206), each of which comprises:
at least one surfactant; and
at least one attachment module;
wherein the attachment modules fit together in at least one conjugate pair so as to assemble the at least two solid cleansing compositions (102, 104, 206) to a composite composition.

2. The composite solid cleansing composition of claim 1, in which an attachment module is plastic or metal.

3. The composite solid cleansing composition of claim 1, in which an attachment module is embedded within each solid cleansing composition (102, 104, 206).

4. The composite solid cleansing composition of claim 3, in which an attachment module comprises at least one hole (704) that is filled with a solid cleansing composition (102, 104, 206).

5. The composite solid cleansing composition of claim 1, in which the solid cleansing compositions (102, 104, 206) fit together in a manner that encloses the attachment modules.

6. The composite solid cleansing composition of claim 1, in which at least one solid cleansing composition (102, 104, 206) comprises at least one additive selected from a polymer, an antibacterial agent, a perfume, a humectant, a dye, a pigment, an exfoliant, a conditioning agent, a plant extract, plant matter, an essential oil, an oil, a wax, a silicone oil, a silicone wax, a chelator, a vitamin, a vitamin derivative, an alkali metal halide, and combinations thereof.

7. The composite solid cleansing composition of claim 6, wherein at least one additive of the composite solid cleansing composition (102, 104, 206) is present in differing amounts in at least two of the solid cleansing compositions (102, 104, 206) that form the composite solid cleansing composition.

8. The composite solid cleansing composition of claim 1, in which at least one of the at least two solid cleansing compositions contains (102, 104, 206) an additive that is absent from at least one of the at least two solid cleansing compositions (102, 104, 206), wherein the additive is selected from an antibacterial agent, a pH-adjusting agent, a perfume, a moisturizing agent, an acne treatment agent, and combinations thereof.

9. The composite solid cleansing composition of claim 1, in which each solid cleansing composition (102, 104, 206) comprises at least one additive selected from a moisturizing agent, an antibacterial agent, an acne treatment agent, and combinations thereof.

10. A method of making a solid composite solid cleansing composition, comprising:
providing at least two solid cleansing compositions (102, 104, 206), each of which comprises an attachment module; and
attaching the at least two solid cleansing compositions (102, 104, 206) through their attachment modules.

11. The method of claim 10, wherein the at least two solid cleansing compositions (102, 104, 206) are attached by a force applied along an attachment axis.

12. The method of claim 10, wherein the at least two solid cleansing compositions (102, 104, 206) are attached by a force applied orthogonal to an attachment axis.

13. A method of making a solid cleansing composition (102, 104, 206) to become part of a composite cleansing composition, comprising:
providing an attachment module, comprising:
a first part that comprises a contact point to another attachment module;
a second part (310) to anchor the attachment module in a solid cleansing composition (102, 104, 206); and
forming a solid cleaning composition (102, 104, 206) around the second part of the attachment module.

14. The method of claim 13, wherein forming a solid cleaning composition (102, 104, 206) around the second part (310) of the attachment module comprises placing the attachment module into a mold and pouring a molten cleansing composition into the mold.

15. The method of claim 13, wherein forming a solid cleansing composition (102, 104, 206) around the second part (310) of the attachment module comprises inserting the attachment module into the solid cleansing composition (102, 104, 206), and pressing the solid cleansing composition (102, 104, 206) to affix the second part (310) of the attachment module to the solid cleansing composition (102, 104, 206).

## Patentansprüche

1. Zusammengesetzte feste Reinigungszusammensetzung, umfassend:
mindestens zwei feste Reinigungszusammensetzungen (102, 104, 206), von denen jede Folgendes umfasst:
mindestens ein Tensid; und
mindestens ein Befestigungsmodul;
wobei die Befestigungsmodule in mindestens einem konjugierten Paar zusammenpassen, um die mindestens zwei festen Reinigungszusammensetzungen (102, 104, 206) zu einer zusammengesetzten Zusammensetzung zusammenzusetzen.

2. Zusammengesetzte feste Reinigungszusammensetzung nach Anspruch 1, in der ein Befestigungsmodul aus Kunststoff oder Metall besteht.

3. Zusammengesetzte feste Reinigungszusammensetzung nach Anspruch 1, in der ein Befestigungsmodul in jede feste Reinigungszusammensetzung (102, 104, 206) eingebettet ist.

4. Zusammengesetzte feste Reinigungszusammensetzung nach Anspruch 3, in der ein Befestigungsmodul mindestens ein Loch (704) umfasst, das mit einer festen Reinigungszusammensetzung (102, 104, 206) gefüllt ist.

5. Zusammengesetzte feste Reinigungszusammensetzung nach Anspruch 1, in der die festen Reinigungszusammensetzungen (102, 104, 206) in einer Weise zusammenpassen, die die Befestigungsmodule umschließt.

6. Zusammengesetzte feste Reinigungszusammensetzung nach Anspruch 1, wobei mindestens eine feste Reinigungszusammensetzung (102, 104, 206) mindestens ein Additiv umfasst, ausgewählt aus einem Polymer, einem antibakteriellen Mittel, einem Parfüm, einem Feuchthaltemittel, einem Farbstoff, einem Pigment, einem Peeling, einem Konditionierungsmittel, einem Pflanzenextrakt, Pflanzenmaterial, einem ätherischen Öl, einem Öl, einem Wachs, einem Silikonöl, einem Silikonwachs, einem Silikonwachs, einem Chelat-Bildner, einem Vitamin, einem Vitaminderivat, einem Alkalimetallhalogenid und Kombinationen davon.

7. Zusammengesetzte feste Reinigungszusammensetzung nach Anspruch 6, wobei mindestens ein Zusatz der zusammengesetzten festen Reinigungszusammensetzung (102, 104, 206) in unterschiedlichen Mengen in mindestens zwei der festen Reinigungszusammensetzungen (102, 104, 206) vorhanden ist, die die zusammengesetzte feste Reinigungszusammensetzung bilden.

8. Zusammengesetzte feste Reinigungszusammensetzung nach Anspruch 1, wobei mindestens eine der mindestens zwei festen Reinigungszusammensetzungen (102, 104, 206) ein Additiv enthält, das in mindestens einer der mindestens zwei festen Reinigungszusammensetzungen (102, 104, 206) nicht vorhanden ist, wobei das Additiv ausgewählt ist aus einem antibakteriellen Mittel, einem pH-Einstellmittel, einem Parfüm, einem Feuchtigkeitsmittel, einem Aknebehandlungsmittel und Kombinationen davon.

9. Zusammengesetzte feste Reinigungszusammensetzung nach Anspruch 1, wobei jede feste Reinigungszusammensetzung (102, 104, 206) mindestens ein Additiv umfasst, ausgewählt aus einem Feuchtigkeitsspender, einem antibakteriellen Mittel, einem Aknebehandlungsmittel und Kombinationen davon.

10. Verfahren zum Herstellen einer festen zusammengesetzten festen Reinigungszusammensetzung, umfassend:
Bereitstellen von mindestens zwei festen Reinigungszusammensetzungen (102, 104, 206), von denen jede ein Befestigungsmodul umfasst; und
Anbringen der mindestens zwei festen Reinigungszusammensetzungen (102, 104, 206) durch ihre Befestigungsmodule.

11. Verfahren nach Anspruch 10, wobei die mindestens zwei festen Reinigungszusammensetzungen (102, 104, 206) durch eine Kraft befestigt werden, die entlang einer Befestigungsachse ausgeübt wird.

12. Verfahren nach Anspruch 10, wobei die mindestens zwei festen Reinigungszusammensetzungen (102, 104, 206) durch eine Kraft orthogonal zu einer Befestigungsachse befestigt werden.

13. Verfahren zur Herstellung einer festen Reinigungszusammensetzung (102, 104, 206), um Teil einer zusammengesetzten Reinigungszusammensetzung zu werden, umfassend: Bereitstellen eines Befestigungsmoduls, umfassend:
ein erstes Teil, das einen Kontaktpunkt zu einem anderen Befestigungsmodul umfasst;
ein zweites Teil (310) zum Verankern des Befestigungsmoduls in einer festen Reinigungszusammensetzung (102, 104, 206); und
Bilden einer festen Reinigungszusammensetzung (102, 104, 206) um den zweiten Teil des Befestigungsmoduls herum.

14. Verfahren nach Anspruch 13, wobei das Bilden einer festen Reinigungszusammensetzung (102, 104, 206) um den zweiten Teil (310) des Befestigungsmoduls das Platzieren des Befestigungsmoduls in einer Form und das Gießen einer geschmolzenen Reinigungszusammensetzung in die Form umfasst.

15. Verfahren nach Anspruch 13, wobei n das Bilden einer festen Reinigungszusammensetzung (102, 104, 206) um den zweiten Teil (310) des Befestigungsmoduls herum das Einsetzen des Befestigungsmoduls in die feste Reinigungszusammensetzung (102, 104, 206) und das Pressen der festen Reinigungszusammensetzung (102, 104, 206) zum Befestigen des zweiten Teils (310) des Befestigungsmoduls an der festen Reinigungszusammensetzung (102, 104, 206) umfasst.

## Revendications

1. Composition composite nettoyante solide, la composition comprenant :
au moins deux compositions nettoyantes solides (102, 104, 206), chacune desquelles comprenant :
au moins un tensioactif ; et
au moins un module de fixation ;
les modules de fixation s'emboîtant ensemble dans au moins une paire conjuguée de manière à assembler les au moins deux compositions nettoyantes solides (102, 104, 206) en une composition composite.

2. Composition composite nettoyante solide selon la revendication 1, dans laquelle un module de fixation est constitué de plastique ou de métal.

3. Composition composite nettoyante solide selon la revendication 1, dans laquelle un module de fixation est incorporé à l'intérieur de chaque composition nettoyante solide (102, 104, 206).

4. Composition composite nettoyante solide selon la revendication 3, dans laquelle un module de fixation comprend au moins un trou (704) qui est rempli d'une composition nettoyante solide (102, 104, 206).

5. Composition composite nettoyante solide selon la revendication 1, dans laquelle les compositions nettoyantes solides (102, 104, 206) s'emboîtent ensemble de manière à confiner les modules de fixation.

6. Composition composite nettoyante solide selon la revendication 1, dans laquelle au moins une composition nettoyante solide (102, 104, 206) comprend au moins un adjuvant choisi parmi un polymère, un agent antibactérien, un parfum, un humectant, un colorant, un pigment, un exfoliant, un agent de conditionnement, un extrait de plante, de la matière végétale, une huile essentielle, une huile, une cire, une huile de silicone, une cire de silicone, un agent de chélation, une vitamine, un dérivé de vitamine, un halogénure de métal alcalin et leurs combinaisons.

7. Composition composite nettoyante solide selon la revendication 6, dans laquelle au moins un adjuvant de la composition composite nettoyante solide (102, 104, 206) est présent en des quantités différentes dans au moins deux des compositions nettoyantes solides (102, 104, 206) qui forment la composition composite nettoyante solide.

8. Composition composite nettoyante solide selon la revendication 1, dans laquelle une composition parmi les au moins deux compositions nettoyantes solides (102, 104, 206) contient un adjuvant qui est absent de l'au moins une composition parmi les au moins deux compositions nettoyantes solides (102, 104, 206), l'adjuvant étant choisi parmi un agent antibactérien, un agent permettant d'ajuster le pH, un parfum, un agent hydratant, un agent destiné au traitement de l'acné et leurs combinaisons.

9. Composition composite nettoyante solide selon la revendication 1, dans laquelle chaque composition nettoyante solide (102, 104, 206) comprend au moins un adjuvant choisi parmi un agent hydratant, un agent antibactérien, un agent destiné au traitement de l'acné et leurs combinaisons.

10. Procédé de fabrication d'une composition composite nettoyante solide, le procédé comprenant :
l'obtention d'au moins deux compositions nettoyantes solides (102, 104, 206), chacune desquelles comprenant un module de fixation ; et
la fixation des au moins deux compositions nettoyantes solides (102, 104, 206) grâce à leur module de fixation.

11. Procédé selon la revendication 10, dans lequel les au moins deux compositions nettoyantes solides (102, 104, 206) sont fixées au moyen d'une force appliquée le long d'un axe de fixation.

12. Procédé selon la revendication 10, dans lequel les au moins deux compositions nettoyante solides (102, 104, 206) sont fixées au moyen d'une force appliquée de manière orthogonale à un axe de fixation.

13. Procédé de fabrication d'une composition nettoyante solide (102, 104, 206) pour faire partie d'une composition nettoyante composite, le procédé comprenant :
l'utilisation d'un module de fixation, le module comprenant :
une première partie qui comprend un point de contact avec un autre module de fixation ;
une seconde partie (310) permettant d'ancrer le module de fixation dans une composition nettoyante solide (102, 104, 206) ; et
la formation d'une composition nettoyante solide (102, 104, 206) autour de la seconde partie du module de fixation.

14. Procédé selon la revendication 13, dans lequel la formation d'une composition nettoyante solide (102, 104, 206) autour de la seconde partie (310) du module de fixation comprend le placement du module de fixation dans un moule et l'introduction d'une composition nettoyante fondue dans le moule.

15. Procédé selon la revendication 13, dans lequel la formation d'une composition nettoyante solide (102, 104, 206) autour de la seconde partie (310) du module de fixation comprend l'insertion du module de fixation dans la composition nettoyante solide (102, 104, 206) et le pressage de la composition nettoyante solide (102, 104, 206) pour coller la seconde partie (310) du module de fixation à la composition nettoyante solide (102, 104, 206).
